Europäisches Patentamt

European Patent Office

Office européen des brevets

(19)

(11) Veröffentlichungsnummer: **0 175 047**
**A2**

(12) **EUROPÄISCHE PATENTANMELDUNG**

(21) Anmeldenummer: 85102366.3

(51) Int. Cl.⁴: **A 61 F 7/00**

(22) Anmeldetag: 02.03.85

(30) Priorität: 18.09.84 DE 8427439 U

(43) Veröffentlichungstag der Anmeldung: 26.03.86
Patentblatt 86/13

(84) Benannte Vertragsstaaten: AT BE CH CH DE FR GB IT LU NL SE

(71) Anmelder: CRIO GmbH Medizintechnik Kirschenmann + Schweizer, Auer Strasse 50, D-7500 Karlsruhe 41 (DE)

(72) Erfinder: Kirschenmann, Günter, Dipl.-Ing. (FH), Buchenstrasse 9, D-7507 Pfinztal-Berghausen (DE)

(74) Vertreter: Lichti, Heiner, Dipl.-Ing. et al, Patentanwälte Dr. Ing. Hans Lichti Dipl.-Ing. Heiner Lichti Dipl.-Phys. Dr.rer.nat. Jost Lempert Durlacher Strasse 31 Postfach 410760, D-7500 Karlsruhe 41 (DE)

(54) **Gerät zur Tieftemperatur-Behandlung rheumatischer Erkrankungen.**

(57) Ein Gerät zur Tieftemperatur-Behandlung rheumatischer Erkrankungen besteht aus einem Flüssiggas-Isolierbehälter (1), einem auf dessen Öffnung aufgesetzten Verschluß (3) mit einem Kühlgaskanal, einem daran angeschlossenen, wärmeisolierten Behandlungsschlauch mit Mundstück und einer Verdampfungseinrichtung für das Flüssiggas, wobei zur Vermeidung von Eisbildung im Behälter durch eintretende Luft und zur Verhinderung des Austretens von Flüssiggaströpfchen aus dem Behälter die Verdampfungseinrichtung aus einer bis in den bodennahen Bereich des Isolierbehälters (1) reichenden, über eine Tauchleitung (11) versorgten Heizung (10) gebildet und vor der dem Isolierbehälter (1) zugekehrten Eintrittsseite des Kühlgaskanals ein Tröpfchen-Separator (13) angeordnet ist.

PATENTANWÄLTE
DR. ING. HANS LICHTI
DIPL.-ING. HEINER LICHTI
DIPL.-PHYS. DR. RER. NAT. JOST LEMPERT

D-7500 KARLSRUHE 41 (GRÖTZINGEN)
DURLACHER STRASSE 31
TEL.: (07 21) 4 85 11

0175047

CRIO GmbH Medizintechnik
Kirschenmann + Schweizer

D-7500 Karlsruhe 41

7578/85

28. Februar 1985

## Gerät zur Tieftemperatur-Behandlung rheumatischer Erkrankungen

- - - - - -

Die Erfindung betrifft ein Gerät zur Tieftemperatur-Behandlung rheumatischer Erkrankungen, bestehend aus einem Flüssiggas-Isolierbehälter, einem auf dessen Öffnung aufgesetzten Verschluß mit einem Kühlgaskanal, einem daran angeschlossenen, wärmeisolierten Behandlungsschlauch mit Mundstück und einer Verdampfungseinrichtung für das Flüssiggas.

Medizinische Untersuchungen aus jüngster Zeit haben ergeben, daß bei rheumatischen Erkrankungen eine Schmerzlinderung und verbesserte Bewegungsfähigkeit, ja sogar eine Heilung dadurch erreicht werden kann, daß die erkrankten Körperpartien mit tiefer Temperatur behandelt werden, wozu Tieftemperaturdämpfe von Flüssiggasen eingesetzt werden, die in Form eines schwachen Strahls auf die zu behandelnden Körperpartien gerichtet werden. Im allgemeinen wird wegen seiner absoluten Ungefährlichkeit und unbegrenzten Mischbarkeit mit der Umgebungsluft flüssiger Stickstoff benutzt, der in einem Isolierbehälter untergebracht ist und aus diesem heraus verdampft wird.

Aus der Praxis sind Geräte bekannt (DE-GM 83 28 806), bei denen die zur Erzielung einer Strömung notwendige erhöhte Verdampfungsrate dadurch erreicht wird, daß unmittelbar an

- 2 -

p

das Flüssiggas erwärmte Luft bzw. Heißluft eingeblasen wird und der Tieftemperaturdampf über eine meist mehrere Meter lange Schlauchleitung entnommen wird. Diese Arbeitsweise setzt voraus, daß vor dem Eintritt der Heißluft in den Behälter ein Trockner angeordnet ist, der die Luftfeuchtigkeit auf ein Minimum reduziert, um eine Eisbildung innerhalb des Behälters oder in den Leitungen zu verhindern. Die Trocknung von Luft bis zu einem Feuchtegehalt von 0% ist nur mit einem außerordentlich hohen apparativen Aufwand möglich, der zu nicht vertretbaren Gestehungskosten für diese Geräte führt. Man muß sich deshalb auf Trockner beschränken, die eine absolute Beseitigung der Luftfeuchtigkeit nicht ermöglichen mit der Folge, daß es zumindest nach einiger Betriebszeit doch zu Vereisungen und Störungen kommt . Ein weiteres Problem liegt darin, daß aus dem "kochenden" Flüssiggas stets Tröpfchen mit in die Dampfatmosphäre gelangen, die dann mit dem Tieftemperaturdampf weggeführt werden. Treten diese Flüssiggaströpfchen an dem Mundstück des Behandlungsschlauchs aus, so können sie auf der Körperoberfläche des Patienten zu Verbrennungen führen. Um dies zu vermeiden, werden lange Behandlungsschläuche verwendet, die zudem nicht geradlinig geführt werden, um die Tröpfchen zur Verdampfung oder aber zur Ablagerung im Behandlungsschlauch und dann zur Verdampfung zu bringen.

Der Erfindung liegt die Aufgabe zugrunde, ein Gerät zu schaffen, bei dem die Verdampfung des Flüssiggases ohne jegliche Eisbildung möglich ist und das Austreten von Flüssiggaströpfchen mit dem Behandlungsstrahl vermieden wird.

Ausgehend von einem Gerät des eingangs geschilderten Aufbaus wird diese Aufgabe zunächst dadurch einer Teillösung zugeführt, daß die Verdampfungseinrichtung aus einer bis in den bodennahen Bereich des Isolierbehälters reichenden , über eine Tauchleitung versorgten Heizung gebildet ist.

Die Erfindung wendet sich also von der bisherigen Arbeitsweise eines direkten Wärmeaustauschs zwischen einem erhitzten Wärmeträger und dem Flüssiggas ab und schlägt statt dessen eine Heizung vor, die in dem Isolierbehälter selbst angeordnet ist und über eine Tauchleitung von außen her mit der notwendigen Energie versorgt wird. Da keine Luft mehr in den Behälter eingebracht wird, entfallen jegliche Eisbildung und die sich daraus ergebenden Funktionsstörungen.

Vorzugsweise ist die Heizung eine elektrische Heizeinrichtung mit Leistungssteuerung. Die Heizeinrichtung kann beispielsweise aus einer Heizpatrone, einem Rohrheizkörper oder einem ähnlichen Widerstandsheizkörper gebildet sein, deren Leistung in einfacher Weise über die Stromversorgung regelbar ist, so daß sich die Abdampfrate ohne Schwierigkeiten einstellen läßt.

Statt dessen kann die Heizung auch aus einem Wärmetauscher mit einem fluiden Wärmeträger gebildet sein, wobei als Wärmeträger Heißluft oder Thermoöle in Frage kommen. Hierbei kommt jedoch der Wärmeträger nicht mit dem Flüssiggas in Berührung, so daß auch hier Funktionsstörungen nicht auftreten können.

Zur weiteren Lösung der Erfindungsaufgabe ist vorgesehen, daß vor der dem Isolierbehälter zugekehrten Eintrittsseite des Kühlgaskanals ein Tröpfchen-Separator angeordnet ist, der für die Abscheidung eventuell mitgerissener Flüssiggastropfen aus der Dampfatmosphäre sorgt, bevor der Tieftemperaturdampf in den Kühlgaskanal und von dort in den Behandlungsschlauch gelangt.

Beispielsweise kann der Tröpfchen-Separator Leitflächen oder Leitkanäle zum Umlenken der Kühlgasströmung aufweisen, wobei eventuell mitgerissene Tröpfchen bei der Umlenkbewegung abgeschieden werden.

In einer vorteilhaften Ausführungsform ist der Tröpfchen-Separator aus einem Rohrstutzen gebildet, der an seinem dem Kühlgaskanal gegenüberliegenden, dem Behälter zugekehrten Ende verschlossen ist und dessen Wandung schrägverlaufende Bohrungen aufweist, deren Eintrittsöffnung höher liegt als die innenseitig im Rohrstutzen mündenden Austrittsöffnungen.

0175047

Der von der Flüssiggas-Oberfläche nach oben abströmende Dampf wird beim Eintritt in die Bohrungen des Rohrstutzens zunächst nach unten und beim Austritt aus den Bohrungen in den Rohrstutzen wiederum nach oben umgelenkt, wobei die Tröpfchen entweder außerhalb oder innerhalb des Rohrstutzens abgeschieden werden. Soweit sie innerhalb des Rohrstutzens abgeschieden werden, gelangen sie auf den verschlossenen Boden des Rohrstutzens und werden dort allmählich verdampft. Hierdurch wird verhindert, daß Flüssigtröpfchen in den Kühlgaskanal und damit in den Behandlungsschlauch gelangen können.

Mit Vorteil ist vorgesehen, daß der Tröpfchen-Separator lediglich in den Hals des Isolierbehälters hineinragt und mit Abstand von der Halswandung angeordnet ist.

Damit findet die Tröpfchenabscheidung am höchsten Punkt des Isolierbehälters und unmittelbar vor dem Eintritt des Dampfs in den Kühlgaskanal statt.

Weitere Einzelheiten und Vorteile der Erfindung ergeben sich aus der nachfolgenden Beschreibung eines in der Zeichnung wiedergegebenen Ausführungsbeispiels. In der Zeichnung zeigen:

Figur 1    eine schematische Ansicht des Gerätes und

Figur 2    einen vergrößerten Detailschnitt im Bereich des Behälterhalses.

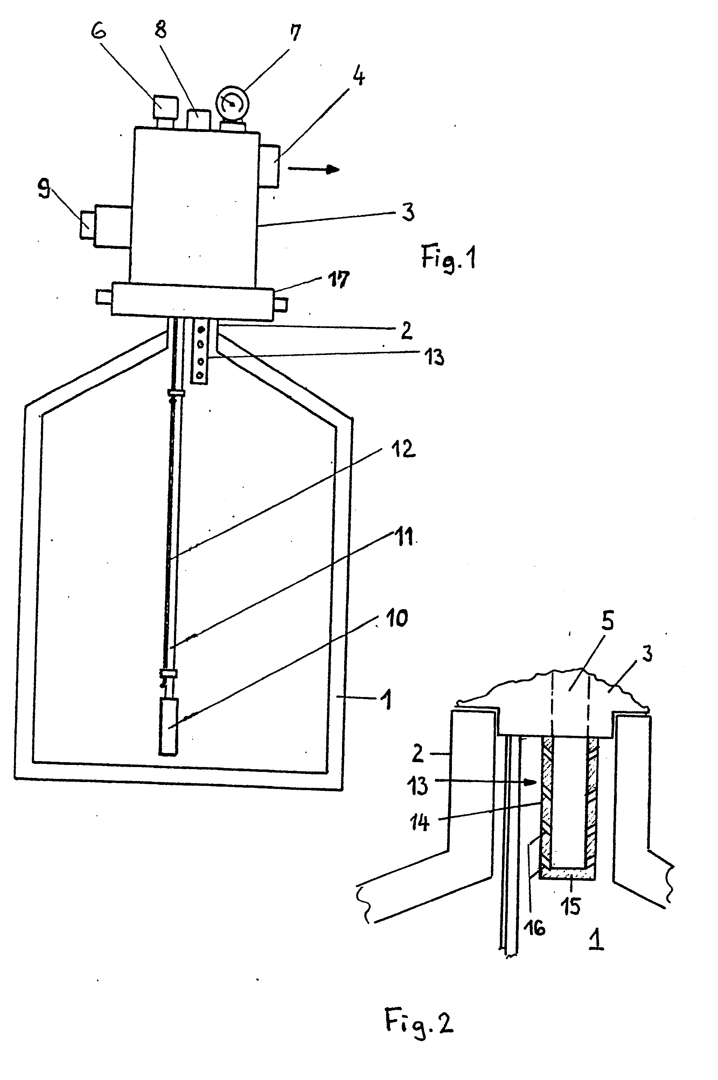

Das in Figur 1 wiedergegebene Behandlungsgerät besteht aus einem Isolierbehälter 1 für ein Flüssiggas, z. B. Flüssig-Stickstoff, einem auf dessen Hals 2 aufgesetzten Verschluß 3 und einem nicht gezeigten, wärmeisolierten Behandlungsschlauch, der an dem Stutzen 4 am Verschluß 3 angeschlossen ist und über den der Tieftemperaturdampf an die Behandlungsstelle gelangt.

0175047

Der nicht gezeigte Behandlungsschlauch weist zu diesem Zweck ein Mundstück für den Austritt des Tieftemperaturdampfs auf. Innerhalb des Verschlusses 3 ist ein Kühlgas- bzw. Tieftemperatur-Dampfkanal 5 angeordnet, über den der Tieftemperaturdampf aus dem Isolierbehälter 1 in den Behandlungsschlauch gelangt.

Der Isolierbehälter 1 weist ferner am Verschluß 3 ein Überdruckventil 6, ein Kontrollmanometer 7 sowie einen Füllstutzen 8 auf. Der Verschluß 3 ist dicht auf den Isolierbehälter 1 aufgeschraubt oder in anderer Weise gasdicht festgelegt. Er kann beispielsweise mittels einer Überwurfmutter 17, die mit einem entsprechenden Außengewinde am Behälterhals 2 zusammenwirkt, befestigt sein. Dadurch läßt sich der Behälter 1 hermetisch abschließen, so daß keine feuchte Luft von außen eindringen kann.

Innerhalb des Behälters 1 befindet sich eine vom Verschluß 3 getragene Heizung 10, die beim wiedergegebenen Ausführungsbeispiel als Heizpatrone ausgebildet und unmittelbar im Bereich des Bodens des Isolierbehälters 1 angeordnet ist. Die Heizung 10 wird über eine Tauchleitung 11 und einen Anschluß 9 am Verschluß 3 mit Strom versorgt. Ferner ist innerhalb des Isolierbehälters 1 ein Füllstandsmesser 12 angeordnet, der den Maximal- und Minimalstand auf einem Steuergerät außerhalb des Isolierbehälters anzeigt.

Vor dem Eintritt des Kühlgaskanals 5 am Verschluß 3 ist ein Tröpfchen-Separator 13 angeordnet, der beim wiedergegebenen Ausführungsbeispiel in den Hals 2 des Isolierbehälters 1 hineinragt. Der Separator besteht beim wiedergegebenen Ausführungsbeispiel aus einem Rohrstutzen 14, der an seinem dem Behälterinneren zugekehrten Ende mit einem Boden 15 verschlossen ist. Die Wandung des Rohrstutzens 14 weist mehrere von außen schräg nach unten und innen verlaufende Bohrungen 16 auf, die den an der Flüssiggas-Oberfläche entstehenden und nach oben strömenden Tieftemperaturdampf innerhalb des Halses 2 zunächst nach unten und dann wieder nach oben umlenken, wobei die Tröpfchen in erster Linie innerhalb des Halses 2 und außerhalb des Rohrstutzens 14 abgeschieden werden.

Der Rohrstutzen 14 wahrt allseitig einen ausreichend großen Abstand von der Innenwandung des Behälterhalses 2 und ist ferner mit einer Vielzahl von Bohrungen 16 ausgestattet, um eine zu starke Drosselung der Dampfströmung zu vermeiden.

PATENTANWÄLTE

DR. ING. HANS LICHTI
DIPL.-ING. HEINER LICHTI
DIPL.-PHYS. DR. RER. NAT. JOST LEMPERT

D-7500 KARLSRUHE 41 (GRÖTZINGEN)
DURLACHER STRASSE 31
TEL.: (07 21) 4 85 11

0175047

7578/85

CRIO GmbH Medizintechnik
Kirschenmann + Schweizer

28. Februar 1985

D-7500 Karlsruhe 41

Patentansprüche

1. Gerät zur Tieftemperatur-Behandlung rheumatischer Erkrankungen, bestehend aus einem Flüssiggas-Isolierbehälter, einem auf dessen Öffnung aufgesetzten Verschluß mit einem Kühlgaskanal, einem daran angeschlossenen, wärmeisolierten Behandlungsschlauch mit Mundstück und einer Verdampfungseinrichtung für das Flüssiggas,
dadurch gekennzeichnet,
daß die Verdampfungseinrichtung aus einer bis in den bodennahen Bereich des Isolierbehälters (1) reichenden, über eine Tauchleitung (11) versorgten Heizung (10) gebildet ist.

2. Gerät nach Anspruch 1, dadurch gekennzeichnet, daß die Heizung (10) eine elektrische Heizeinrichtung mit Leistungssteuerung ist.

3. Gerät nach Anspruch 1, dadurch gekennzeichnet, daß die Heizung (10) aus einem Wärmetauscher mit einem fluiden Wärmeträger gebildet ist.

4. Gerät nach einem der Ansprüche 1 bis 3, dadurch gekennzeichnet, daß vor der dem Isolierbehälter (1) zugekehrten Eintrittsseite des Kühlgaskanals (5) ein Tröpfchen-Separator (13) angeordnet ist.

- 2 -

p

5. Geräte nach einem der Ansprüche 1 bis 4, dadurch gekennzeichnet, daß der Tröpfchen-Separator (13) Leitflächen oder Leitkanäle (16) zum Umlenken der Kühlgasströmung aufweist.

6. Gerät nach einem der Ansprüche 1 bis 5, dadurch gekennzeichnet, daß der Tröpfchen-Separator (13) aus einem Rohrstutzen (14) gebildet ist, der an seinem dem Kühlgaskanal (5) gegenüberliegenden, dem Behälter (1) zugekehrten Ende verschlossen ist und dessen Wandung schräg verlaufende Bohrungen (16) aufweist, deren Eintrittsöffnung höher liegt als die innenseitig im Rohrstutzen (14) mündenden Austrittsöffnungen.

7. Gerät nach einem der Ansprüche 1 bis 6, dadurch gekennzeichnet, daß der Tröpfchen-Separator (13) lediglich in den Hals (2) des Isolierbehälters (1) hineinragt und mit Abstand von der Halswandung angeordnet ist.

1/1

0175047

Fig.1

Fig.2